(19) **Europäisches Patentamt**
**European Patent Office**
**Office européen des brevets**

(11) **EP 2 364 174 B1**

(12) **FASCICULE DE BREVET EUROPEEN**

(45) Date de publication et mention de la délivrance du brevet:
**18.01.2017 Bulletin 2017/03**

(51) Int Cl.:
***A61M 16/00*** *(2006.01)*

(21) Numéro de dépôt: **09768133.2**

(22) Date de dépôt: **03.11.2009**

(86) Numéro de dépôt international:
**PCT/FR2009/052121**

(87) Numéro de publication internationale:
**WO 2010/061092 (03.06.2010 Gazette 2010/22)**

(54) **SYSTÈME DE QUANTIFICATION DU DESACCORD ENTRE UN PATIENT SOUS ASSISTANCE RESPIRATOIRE ET UN APPAREIL D'ASSISTANCE CORRESPONDANT**

SYSTEM ZUR QUANTIFIZIERUNG DER DISKREPANZ ZWISCHEN EINEM PATIENTEN MIT ATEMUNTERSTÜTZUNG UND EINER ENTSPRECHENDEN UNTERSTÜTZUNGSVORRICHTUNG

SYSTEM FOR QUANTIFYING THE DISCREPANCY BETWEEN A PATIENT RECEIVING ASSISTED BREATHING AND A CORRESPONDING ASSISTANCE DEVICE

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **03.11.2008 FR 0857451**

(43) Date de publication de la demande:
**14.09.2011 Bulletin 2011/37**

(73) Titulaire: **Assistance Publique Hôpitaux De Paris**
**75001 Paris (FR)**

(72) Inventeurs:
 • HEYER, Laurent
  75010 Paris (FR)
 • BACONNIER, Pierre
  38000 Grenoble (FR)

(74) Mandataire: **Lavoix**
**2, place d'Estienne d'Orves**
**75441 Paris Cedex 09 (FR)**

(56) Documents cités:
**WO-A1-2008/116318    WO-A2-2004/002561**

**Description**

La présente invention concerne un système de quantification du désaccord entre un patient sous assistance respiratoire et un appareil d'assistance correspondant.

Ce système s'inscrit dans le cadre de recherches sur l'optimisation des stratégies et des méthodes de surveillance et d'assistance de la fonction respiratoire en anesthésie et en réanimation.

Les progrès en anesthésie et en réanimation ont pour objectifs la réduction de la durée de la surveillance et l'amélioration de la qualité de la récupération du patient.

Une assistance respiratoire doit assurer une ventilation efficace et non délétère avec un confort acceptable pour le patient.

Dans ce contexte, l'accord entre un patient et l'appareil d'assistance respiratoire correspondant est déterminant. En situation clinique la détection d'un éventuel désaccord est essentielle pour l'optimisation de la stratégie thérapeutique.

Dans l'état de la technique, la surveillance de l'interaction entre le patient et son appareil d'assistance se heurte à la difficulté d'estimer correctement l'activité respiratoire du patient de façon robuste et non invasive.

En effet, les dispositifs non invasifs actuels sont régulièrement pris en défaut. Les limitations de ces dispositifs qui sont actuellement implémentés dans les appareils d'assistance, sont responsables d'un défaut de détection de l'activité inspiratoire du patient qui cause un désaccord entre le patient et l'appareil d'assistance et qui se traduit par une assistance sous optimale.

Les dispositifs alternatifs actuellement disponibles dans l'état de la technique nécessitent des capteurs de mesure de l'activité respiratoire musculaire qui sont à la fois invasifs (pression intra-thoracique, électromyographie aiguille...) et peu robustes soit en raison des perturbations physiologiques ou de l'évolution clinique du patient soit en raison de la durée de vie du capteur pour assurer une surveillance continue.

Par ailleurs, il n'existe pas dans l'état de la technique de système permettant de délivrer à un opérateur, une information de quantification du désaccord entre le patient et l'appareil d'assistance correspondant de façon simple et qui soit directement perceptible par l'opérateur, afin d'engager un processus de correction adapté si nécessaire. WO2004/002561 divulgue un système adapté à la quantification du désaccord entre un patient sous assistance respiratoire et un appareil d'assistance.

Le but de l'invention est donc de résoudre ces problèmes.

A cet effet, l'invention a pour objet un système de quantification du désaccord entre un patient sous assistance respiratoire et un appareil d'assistance correspondant, caractérisé en ce qu'il comporte :

- des moyens d'acquisition d'informations relatives à l'état respiratoire du patient sur un cycle respiratoire,
- des moyens d'acquisition d'informations relatives à l'état de fonctionnement de l'appareil d'assistance sur un cycle mécanique,
- des moyens de filtrage et de traitement de ces informations pour détecter et localiser les changements d'états du patient sur un cycle respiratoire et de l'appareil sur un cycle mécanique,
- des moyens de calcul d'une information de désynchronisation entre le patient et l'appareil à partir de ces changements d'états, et
- des moyens formant interface homme/machine de restitution de cette information de désynchronisation à un opérateur.

Selon d'autres aspects de l'invention, le système de quantification comprend l'une ou plusieurs des caractéristiques suivantes :

- les moyens d'acquisition des informations relatives à l'état respiratoire du patient comprennent des moyens d'acquisition de signaux de pression et de débit de l'air dans un circuit pneumatique entre le patient et l'appareil d'assistance, à destination de moyens d'estimation en continu de la pression théorique de l'air attendue dans le circuit pneumatique en l'absence d'activité musculaire respiratoire du patient et des moyens de comparaison des pressions théorique estimée et mesurée pour détecter en continu une différence de pression représentative d'une activité musculaire respiratoire du patient,
- les moyens d'acquisition d'informations relatives à l'état de fonctionnement de l'appareil d'assistance comprennent des moyens de traitement d'un signal de débit d'air de l'appareil pour détecter les états d'exhalation et d'insufflation de celui-ci,
- les moyens d'acquisition d'informations relatives à l'état de fonctionnement de l'appareil d'assistance sont intégrés dans celui-ci,
- les moyens d'acquisition d'informations relatives à l'état de fonctionnement de l'appareil d'assistance comprennent un capteur de débit d'air associé au circuit pneumatique entre l'appareil d'assistance et le patient,
- les moyens de calcul de l'information de désynchronisation comprennent des moyens de calcul d'un score de désaccord patient/machine selon la relation :

$$QI(N) = H(N) - [H(B) + H(C)]$$

dans laquelle QI(N) est le score de désynchronisation, H(N) est l'entropie de Shannon du système appareil d'assistance + patient, H(B) est l'entropie de Shannon du patient seul et H(C) est l'entropie de Shannon de l'appareil d'assistance seul,
- les moyens formant interface homme/machine comprennent des moyens d'affichage de ce score,
- les moyens d'affichage comprennent des moyens d'affichage de ce score sous forme numérique,
- les moyens d'affichage comprennent des moyens d'affichage de ce score sous forme graphique,
- les moyens de calcul sont adaptés pour calculer l'information de désynchronisation sur un nombre de cycles respiratoires prédéterminé,
- les moyens d'estimation comprennent des moyens paramétrables et adaptatifs de modélisation du système respiratoire passif du patient,
- les moyens de modélisation se présentent sous la forme de modèles fonction au moins du volume et du débit d'air circulant dans le circuit pneumatique.
- les moyens de modélisation comprennent un ensemble de modèles paramétrables et en ce que les moyens d'estimation comprennent des moyens d'extraction du signal de pression mesurée, de paramètres d'entrée de ces modèles, de façon à déclencher le fonctionnement de ces modèles sur la base de ces paramètres et des moyens de sélection du modèle le plus discriminant en terme de détection et de non-détection d'activité musculaire respiratoire du patient et/ou le plus simple en terme de nombre de paramètres utilisés, pour retenir son estimation,
- les moyens d'extraction des paramètres sont adaptés pour extraire les paramètres sur au moins un cycle mécanique composé successivement d'une insufflation et d'une exhalation, en excluant la phase de pressurisation au début du cycle mécanique courant et la phase de déclenchement de l'insufflation du cycle suivant, à la fin du cycle mécanique courant,
- la phase de pressurisation et la phase de déclenchement de l'insufflation sont détectées par des moyens d'analyse de la pression et du débit d'air dans le circuit pneumatique,
- les moyens d'analyse sont raccordés aux moyens d'acquisition des signaux de pression et de débit dans le circuit pneumatique,
- les moyens d'analyse sont intégrés dans l'appareil d'assistance,
- les phases de pressurisation et de déclenchement de l'insufflation sont détectées par des moyens d'analyse à partir d'un signal complémentaire délivrant une information physiologique liée à l'activité musculaire respiratoire du patient, et
- le signal complémentaire est un signal d'électromyogramme.

Ainsi, un système selon l'invention permet d'assurer une détection de l'activité musculaire respiratoire d'un patient sous assistance respiratoire, d'une part à partir de mesures non invasives et déjà disponibles la plupart du temps avec des appareils d'assistance respiratoire actuels, et d'autre part avec une méthode qui permet de contourner les limitations liées à l'évolution de l'état clinique du patient, des méthodes déjà connues.

De plus, un tel système permet à travers des moyens formant interface homme-machine de restituer de façon simple et directement perceptible par l'opérateur, une information de désynchronisation pour lui permettre d'engager les démarches d'adaptation nécessaires.

L'invention sera mieux comprise à l'aide de la description qui va suivre, donnée uniquement à titre d'exemple et faite en se référant aux dessins annexés, sur lesquels :

- la figure 1 représente un schéma synoptique illustrant la structure et le fonctionnement de moyens d'acquisition d'informations relatives à l'état respiratoire d'un patient entrant dans la constitution d'un système de quantification selon l'invention,
- la figure 2 représente un schéma synoptique illustrant la structure et le fonctionnement de moyens d'estimation de pression entrant dans la constitution des moyens d'acquisition d'informations relatives à l'état respiratoire du patient,
- la figure 3 illustre un cycle mécanique d'insufflation et d'exhalation,
- la figure 4 représente un schéma synoptique illustrant la structure et le fonctionnement d'un premier mode de réalisation d'un système de quantification selon l'invention,
- la figure 5 représente un schéma synoptique illustrant la structure et le fonctionnement d'un second mode de réalisation d'un système de quantification selon l'invention, et
- la figure 6 illustre l'évolution d'un désaccord patient-appareil d'assistance.

L'invention concerne donc un système de quantification du désaccord entre un patient sous assistance respiratoire et un appareil d'assistance correspondant.

Ce système comporte des moyens d'acquisition d'informations relatives à l'état respiratoire du patient, des moyens d'acquisition d'informations relatives à l'état de fonctionnement de l'appareil d'assistance, des moyens de filtrage et de traitement de ces informations pour détecter et localiser les changements d'états du patient et de l'appareil, des moyens de calcul d'une information de désynchronisation entre le patient et l'appareil à partir de ces changements d'états et des moyens formant interface homme-machine de restitution de cette information de désynchronisation à un opérateur.

Ce système vise alors à mettre à disposition de l'opérateur un score permettant de qualifier, sous forme symbolique et interprétable par celui-ci, la qualité de l'accord patient-appareil. Ce score se base sur une analyse statistique des cycles respiratoires présentant une désynchronisation parmi un nombre de cycles donné.

Le principe du système de quantification repose sur le calcul de l'entropie de Shannon du système global patient-appareil et des sous-systèmes dissociés patient d'une part et appareil d'autre part. Le score de désynchronisation quantifie alors le désaccord entre le patient et son appareil d'assistance par la quantité d'informations supplémentaires générées par l'observation d'un système pris dans sa globalité par rapport à l'information générée par l'ensemble des sous-systèmes composant ce système global à savoir d'une part le patient et d'autre part l'appareil. Ce gain d'informations reflète alors l'information générée par l'interaction entre les sous-systèmes étudiés.

Ainsi, le système de quantification selon l'invention comporte donc des moyens d'acquisition d'informations relatives à l'état respiratoire du patient.

Ces moyens d'acquisition d'informations relatives à l'état respiratoire du patient reposent sur une détection / calcul adaptatif d'une pression musculaire représentative de l'activité musculaire respiratoire de ce patient sous assistance respiratoire.

La pression musculaire peut être détectée ou calculée à partir de signaux de débit et de pression mesurés dans le circuit pneumatique qui relie le patient à l'appareil d'assistance.

A chaque cycle mécanique c'est-à-dire de l'appareil, les paramètres d'un modèle mécanique du système respiratoire passif du patient sont identifiés sur des plages prédéterminées du cycle respiratoire à partir du signal de débit, afin d'estimer, sur l'ensemble du cycle mécanique, la pression théorique attendue en l'absence d'activité musculaire du patient. La différence arithmétique entre cette pression théorique et la pression mesurée est représentative de la pression générée par l'activité musculaire respiratoire du patient et est appelée pression musculaire (Pmus). L'écart à zéro de cette pression indique une activité musculaire respiratoire qui est inspiratoire ou expiratoire selon le signe de cet écart. Par ce moyen, les cycles respiratoires du patient sont identifiés, un cycle respiratoire comprenant une expiration et une inspiration complètes.

Grâce à un tel système, il est possible d'adapter automatiquement les paramètres de calcul de la pression musculaire à la fois aux spécificités mécaniques du système respiratoire passif du patient et aux particularités de son comportement respiratoire de sorte que la détection de l'activité respiratoire musculaire soit en continu la plus optimale possible.

Ce système utilise les connaissances sur les conditions de déclenchement des insufflations pour adapter les paramètres du calcul de la pression musculaire, c'est-à-dire le choix du modèle de la mécanique du système respiratoire passif, la définition des zones d'identification des paramètres de ce modèle et le choix des seuils de détection d'une activité musculaire au cours du cycle mécanique.

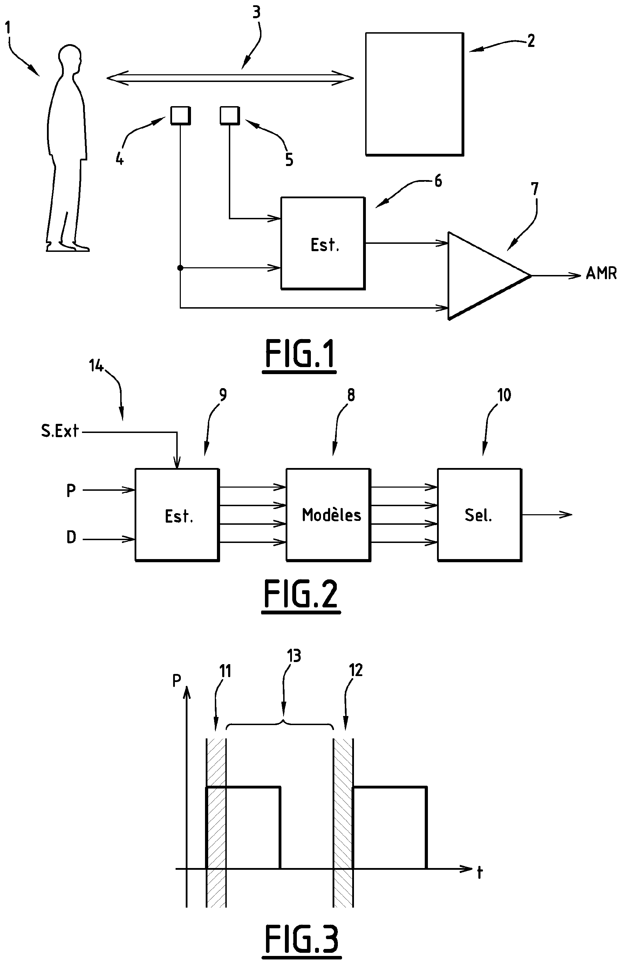

Un tel système est illustré schématiquement sur la figure 1.

Sur cette figure 1, le patient est désigné par la référence générale 1 et l'appareil d'assistance respiratoire est désigné par la référence générale 2, le patient et l'appareil respiratoire étant reliés par un circuit pneumatique désigné par la référence générale 3, de façon classique.

Dans l'exemple de réalisation illustré sur cette figure, le circuit pneumatique 3 est associé à des moyens d'acquisition de signaux de pression et de débit de l'air dans ce circuit, ces moyens étant désignés par les références 4 et 5 respectivement.

Ces moyens d'acquisition 4 et 5 sont alors adaptés pour délivrer ces signaux à des moyens d'estimation en continu de la pression théorique de l'air attendue dans le circuit pneumatique en l'absence d'activité musculaire respiratoire du patient.

Ces moyens d'estimation sont désignés par la référence générale 6 sur cette figure 1 et sont basés sur l'utilisation de moyens paramétrables et adaptatifs de modélisation du système respiratoire passif du patient comme cela sera décrit plus en détail par la suite.

Ces moyens d'estimation 6 délivrent alors une information de pression théorique estimée à des moyens de comparaison désignés par la référence générale 7, recevant sur une autre entrée la pression réellement mesurée dans le circuit pneumatique, ce qui permet de détecter en continu une différence de pression représentative d'une activité musculaire respiratoire du patient.

En fait, et comme cela a été indiqué précédemment, la différence arithmétique entre cette pression théorique estimée et la pression mesurée est représentative de la pression générée par l'activité musculaire respiratoire du patient et est appelée pression musculaire Pmus. L'écart à zéro de cette pression indique une activité musculaire respiratoire qui est inspiratoire ou expiratoire selon le signe de cet écart.

Comme cela est illustré sur la figure 2, les moyens de modélisation des moyens d'estimation comprennent un ensemble de modèles paramétrables de systèmes respiratoires passifs de patient, désigné par la référence générale 8 sur cette figure. De tels modèles sont déjà bien connus dans l'état de la technique et permettent de modéliser le comportement de la mécanique du système respiratoire du patient comme cela sera décrit plus en détail par la suite.

Ces modèles sont paramétrables, et les moyens d'estimation comprennent alors des moyens d'extraction du signal de pression mesurée, de paramètres d'entrée de ces modèles de façon à déclencher le fonctionnement de ces modèles sur la base de ces paramètres. Ces moyens d'extraction sont désignés par la référence générale 9 sur cette figure 2 et leur fonctionnement sera décrit plus en détail également par la suite.

Les moyens d'estimation 6 comprennent également des moyens de sélection du modèle le plus discriminant en terme de détection et de non détection d'activité musculaire respiratoire du patient et/ou le plus simple en terme de nombre de paramètres utilisés, pour retenir son estimation, ces moyens de sélection étant désignés par la référence générale 10.

En fait, et comme cela est illustré par exemple sur la figure 3, les moyens d'extraction 9 des paramètres sont adaptés pour extraire les paramètres d'au moins un cycle mécanique composé successivement d'une insufflation et d'une exhalation par exemple en excluant la phase de pressurisation au début du cycle mécanique courant et la phase de déclenchement de l'insufflation du cycle suivant, à la fin du cycle mécanique courant.

Ceci est illustré par les zones hachurées sur la figure 3 où l'on a représenté des cycles mécaniques successifs. La zone hachurée désignée par la référence générale 11 sur cette figure 3 correspond à la phase de pressurisation au début du cycle mécanique courant tandis que la zone hachurée désignée par la référence générale 12, correspond à la phase de déclenchement de l'insufflation du cycle suivant, à la fin du cycle mécanique courant.

L'extraction des paramètres des modèles se fait alors sur la zone désignée par la référence générale 13 entre ces deux zones d'exclusion.

Bien entendu différentes détections de ces phases sont possibles. C'est ainsi par exemple que ces phases sont détectées par des moyens d'analyse de la pression et du débit d'air dans le circuit pneumatique, les moyens d'analyse étant alors raccordés aux moyens d'acquisition des signaux de pression et de débit dans le circuit pneumatique tel que décrit précédemment.

Cependant les moyens d'analyse peuvent également être intégrés dans l'appareil d'assistance directement.

De même, les phases de pressurisation et de déclenchement de l'insufflation peuvent également être détectées par des moyens d'analyse à partir d'un signal complémentaire délivrant une information physiologique liée à l'activité musculaire respiratoire du patient tel que par exemple un signal complémentaire d'électromyogramme de surface comme désigné par la référence générale 14 sur la figure 2.

Ce système permet alors d'adapter les paramètres de calcul de la pression musculaire, c'est-à-dire de choisir le modèle de la mécanique du système respiratoire passif du patient, de définir les zones d'identification des paramètres de ce modèle et le seuil de détection d'une activité musculaire.

Il s'agit alors de sélectionner de façon adaptative, dans un jeu de différents modèles hiérarchiques et de complexité croissante, écrits sous une forme par exemple linéaire adaptée à l'identification des paramètres du modèle, un modèle adapté, par la méthode de régression linéaire multiple et au sens des moindres carrés, de façon classique.

Le modèle le plus simple, dit de référence, est une forme linéaire avec quatre paramètres $P=f(V,D)=P_o+(V_o)*(V+(R_o+Rd*D)*D)$ avec V et D correspondant aux signaux de volume et de débit d'air en fonction du temps. Le volume est calculé à partir du signal D de débit par intégration en fonction du temps. Ce modèle de référence permet une détection efficace des activités inspiratoires qui déclenchent une insufflation avec une identification cycle à cycle mécanique de ses paramètres. Cette identification est réalisée à partir des signaux de débit et de volume d'air correspondant à des périodes du cycle mécanique non affectées par les phénomènes mécaniques non décrits par le modèle et non concernées par une activité musculaire inspiratoire qui déclenche une insufflation. Cette identification est efficace alors même que ces zones d'identification sont fixes d'un patient à l'autre et indépendante du comportement respiratoire du patient. Cette zone d'identification peut comporter deux parties disjointes, l'une au cours de la phase d'insufflation et l'une autre au cours de la phase d'exhalation du cycle mécanique. Toujours pour ce modèle de référence, un seuil de détection d'une activité musculaire respiratoire fixe qui est compris entre 0,5 et 2 cmH2O et de préférence égal à 1 cmH2O permet une détection efficace d'une activité inspiratoire qui déclenche une insufflation.

D'autres modèles comportent n-k paramètres avec n > k et (n-k) > 4 et sont également de formes linéaires f(V,D,A) avec V,D et A qui correspondent aux signaux de volume, de débit et d'accélération de l'air en fonction du temps. L'accélération est la dérivée première du signal D de débit. Ces modèles plus complexes ont l'avantage d'avoir la capacité de décrire des phénomènes mécaniques qui ne le sont pas par le modèle de référence comme la transition entre la fin de l'insufflation et le début de l'exhalation du cycle mécanique.

Cette capacité permet de proposer une zone d'identification non plus disjointe mais continue, à la fois sur l'insufflation et l'exhalation. Cela présente deux avantages :

1) Une définition plus simple de la zone d'identification par l'exclusion d'une période à la fin d'une exhalation (ou juste avant l'insufflation) définie par un délai télé-exhalation (Dte) et d'une période au début de l'insufflation définie par un délai proto-insufflation (Dpi) ; et

2) Une amélioration de l'identification des paramètres des modèles par la prise en compte de la période de transition entre l'insufflation et l'exhalation caractérisée par des variations importantes du débit et de sa dérivée.

Néanmoins, l'augmentation de la complexité du modèle et l'extension de sa capacité à décrire des phénomènes mécaniques plus complexes induisent potentiellement une réduction de la sensibilité de la détection. Schématiquement, tout se passe comme si des phénomènes transitoires liés à une activité musculaire sont alors attribués aux caractéristiques mécaniques du système respiratoire passif.

Le système selon l'invention utilise une méthode de sélection des paramètres de calcul de la pression musculaire qui permet d'adapter le modèle et la zone d'identification de ses paramètres pour assurer une détection efficace de l'activité musculaire respiratoire sur l'ensemble du cycle mécanique.

La dégradation de la performance de l'identification de l'activité musculaire respiratoire à partir du calcul de la pression musculaire avec ces modèles plus complexes peut être efficacement compensée par une sélection appropriée du modèle mécanique et de la zone d'identification des paramètres du modèle :

- le modèle sélectionné doit être le plus proche possible de la mécanique effective du système respiratoire passif du patient ;
- la zone d'identification des paramètres doit être adaptée au comportement respiratoire du patient de telle sorte que la période du cycle mécanique exclue soit la plus proche possible de la période où le patient a une activité inspiratoire ;
- le seuil de détection de l'activité respiratoire doit être adapté en fonction de la qualité d'ajustement du modèle sélectionné et des paramètres identifiés.

Le principe de l'adaptation consiste à comparer le résultat de l'identification des activités inspiratoires qui déclenchent une insufflation pour un jeu de différents modèles et délais (Dte et Dpi) et à sélectionner les paramètres les plus adaptés sur trois critères :

1 - la capacité à assurer une détection correcte des activités connues : La détection des insufflations détectées par une activité inspiratoire (Ct-(n-k)) ou d'une activité inspiratoire qui déclenche une insufflation (Ait-(n-k)) et qui doit être identique à la détection de référence (Ct-ref).

2 - les caractéristiques d'une inspiration qui déclenche une insufflation. La zone active sélectionnée est la zone la plus courte qui précède l'insufflation et dont la durée totale (Dte + Dpi) est supérieure ou égale à la durée minimale d'une activité inspiratoire considérée comme significative (celle-ci est de l'ordre de plusieurs dixièmes de seconde et de préférence égale à 0.3 seconde).

3 - les propriétés des modèles hiérarchiquement emboîtés : le modèle sélectionné est le modèle le plus simple assurant un ajustement sur la zone d'identification des paramètres de la pression mesurée qui est statistiquement équivalent au modèle le plus complexe (ou comportant le plus de paramètres).

Pour chaque combinaison de modèles et de zones d'identification, le résultat de la détection d'une insufflation déclenchée (Ct-(n-k)) est calculé sur une période de plusieurs dizaines de cycles mécaniques (de préférence 20) et est comparé au résultat de référence (Ct-ref). Parmi les combinaisons dont le résultat est superposable au résultat de référence (Critère 1), les combinaisons correspondant à la zone d'exclusion optimale (Critère 2) sont identifiées et ensuite parmi ces combinaisons, le modèle optimal est sélectionné (Critère 3).

Cette adaptation automatique du modèle permet d'assurer d'une part une détection des activités qui déclenchent une insufflation au moins aussi efficace que la méthode de référence validée et d'autre part une détection efficace des autres activités respiratoires présentes au cours d'un cycle mécanique en cas de désadaptation de l'activité respiratoire du patient et de son appareil d'assistance.

Cette méthode présente de plus l'avantage de pouvoir suivre en continu, à la fois l'évolution du comportement respiratoire et de la mécanique du système respiratoire du patient.

Selon un premier mode de réalisation, le dispositif utilise la détection des insufflations déclenchées par l'inspiration du patient par le modèle de référence avec quatre paramètres. Cette réalisation présente l'avantage de réduire les signaux d'entrée du dispositif aux seuls signaux de pression et de débit.

Selon un deuxième mode de réalisation, l'information sur le mécanisme de déclenchement des insufflations, est fournie par un signal additionnel qui peut être fourni par l'appareil d'assistance ou par un capteur additionnel. Dans le premier cas, il s'agit d'un signal représentatif de l'état d'ouverture et de déclenchement des valves d'insufflation internes à l'appareil d'assistance. Dans le deuxième cas, il s'agit d'un signal représentatif de l'activité inspiratoire du patient fourni par un capteur non-invasif et distinct des signaux de pression ou de débit comme un détecteur d'activité d'un muscle avec une activité inspiratoire à partir d'un électromyogramme de surface (sEMG ou sMMG) ou encore de mouvement (impédencemétrie).

Ce système automatique de détection a été testé expérimentalement dans trois situations :

- i) pour évaluer la méthode de sélection du modèle le plus simple adapté (Critère 3) ;
- ii) pour évaluer la méthode de définition optimale des zones d'identification (Critère 2) ;
- iii) pour évaluer la combinaison de ces méthodes.

1) Evaluation de la méthode de sélection du modèle le plus simple adapté (critère 3) :

Cette étude a été réalisée à partir d'enregistrements de pression et de débit réalisés sur un poumon mécanique artificiel ventilé par un appareil d'assistance dont les caractéristiques mécaniques sont parfaitement connues. La méthode de sélection sur la qualité de l'ajustement mesurée par la comparaison statistique du résidu conduit à sélectionner comme modèle le plus simple statistiquement équivalent (avec un risque alpha de 1% ou 5%) le modèle minimum nécessaire pour décrire la mécanique du poumon mécanique.

2) Evaluation de la méthode de définition optimale des zones d'identification (critère 2) :

Cette étude a été réalisée à partir d'enregistrements anciens réalisés chez 14 patients sous assistance respiratoire partielle qui avaient subi un échec de sevrage bien conduit et pour cela redevables d'une exploration spécifique et invasive de leur activité respiratoire par la mesure de la pression oesophagienne.

Chez ces patients, la capacité de détection par la méthode non invasive est mesurée par la concordance entre l'activité détectée par le calcul de la pression musculaire et l'activité détectée par la lecture de la pression oesophagienne. La valeur de la concordance calculée avec la méthode automatique de sélection des zones d'identification est comparée par la méthode de Bland et Altman à la concordance optimale observée pour l'ensemble des combinaisons possibles de délais qui définissent la zone d'identification et pour un modèle complexe fixe. L'analyse de la représentation graphique permet de dire que les deux méthodes sont interchangeables avec une réduction de la concordance avec la méthode automatique faible de 4% et un écart moyen de 4%.

Chez ces patients, les valeurs de délais optimaux de définition de la zone d'exclusion ont été identifiées pour la détection des activités qui déclenchent une insufflation et pour une détection de l'ensemble des activités inspiratoires déclenchantes ou non. Ces délais optimaux et en particulier le délai télé-exhalation (Dte) sont directement liés aux délais mesurés entre le début d'activité inspiratoire et le déclenchement de l'insufflation défini à partir de la pression oesophagienne.

3) Evaluation de la méthode de définition optimale :

Cette étude a été réalisée à partir d'enregistrements anciens réalisés chez 17 patients sous assistance respiratoire partielle redevables d'une exploration non-invasive de leur activité respiratoire par la mesure d'un électromyogramme de surface du diaphragme (sEMG). La détection des activités inspiratoires déclenchantes et non par la méthode automatique est comparée par la méthode de Bland et Altman à celle fournie par la lecture manuelle des signaux sEMG, Débit et Pression. Ces deux méthodes sont superposables pour la détection des deux types d'activités inspiratoires qui déclenchent et qui ne déclenchent pas une insufflation.

Il est bien entendu que différents modes de réalisation d'un tel système de détection de l'activité respiratoire du patient peuvent encore être envisagés.

Ainsi, cette information relative à l'état respiratoire du patient peut être obtenue par d'autres moyens.

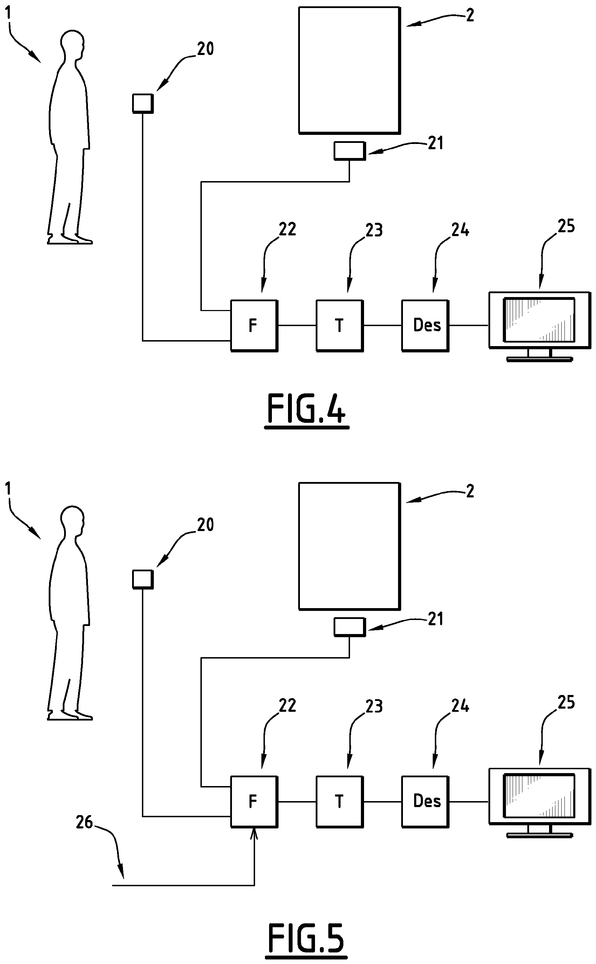

Ces moyens d'acquisition sont désignés par la référence générale 20 sur les figures 4 et 5, cette référence désignant de façon générale tous types de moyens d'acquisition d'informations relatives à l'état respiratoire du patient 1.

Sur ces figures 4 et 5, l'appareil d'assistance est toujours désigné par la référence générale 2. Cet appareil d'assistance est lui aussi associé à des moyens d'acquisition d'informations relatives à son état de fonctionnement.

Ces moyens d'acquisition sont désignés par la référence générale 20 sur ces figures 4 et 5 et peuvent comporter des moyens d'analyse et de traitement d'un signal de débit d'air de l'appareil pour détecter les états d'exhalation et d'insufflation, à partir d'un capteur de débit correspondant associé au circuit pneumatique 3. Ces moyens peuvent également être intégrés à l'appareil.

En fait, ces moyens sont destinés à détecter le début et la fin des différents états de l'activité respiratoire du patient ou cycle respiratoire et des différents états d'activité de l'appareil d'assistance ou cycle mécanique. Ceci permet alors d'analyser les activités du patient et de l'appareil, décrits tous les deux sous la forme de moteurs discrets, caractérisés par différents états discrets.

Pour le patient ces états sont habituellement réduits à des états d'inspiration, d'expiration et d'expiration active. Pour l'appareil d'assistance, ces états sont des états d'insufflation et d'exhalation. De plus, les moyens d'acquisition d'informations relatives à l'état de fonctionnement de l'appareil délivrent également un signal représentatif des mécanismes d'arrêt du cycle mécanique.

Ceux-ci sont en effet soit déclenchés soit non déclenchés par l'activité respiratoire musculaire du patient.

Ces informations relatives au patient et à l'appareil sont ensuite transmises à des moyens de filtrage et de traitement numérique en temps réel pour permettre un débruitage et une localisation des débuts et des fins de changements d'états de l'appareil et du patient, ces moyens étant désignés par les références générales 22 et 23 respectivement sur ces figures 4 et 5.

Ces moyens sont raccordés à des moyens de calcul de l'information de désynchronisation entre le patient et l'appareil à partir de ces changements d'états, ces moyens de calcul étant désignés par les références générales 24 sur ces figures 4 et 5.

Ceux-ci délivrent alors après calcul une information de désynchronisation à des moyens formant interface homme-machine de restitution de celle-ci à un opérateur, ces moyens formant interface homme-machine étant désignés par la référence générale 25 sur ces figures 4 et 5.

Cette information est alors représentative de la désynchronisation entre l'appareil respiratoire et le patient et permet de quantifier ce désaccord de façon perceptible pour un opérateur.

Dans le système selon l'invention, les informations sur les débuts et les fins des cycles mécanique et respiratoire sont comparées pour définir les mécanismes d'arrêt d'un état du cycle mécanique, afin de classer les interactions définies pour chaque cycle respiratoire ou mécanique.

Quatre classes peuvent ainsi être définies :

- arrêt de l'insufflation et de l'exhalation activement déclenché,
- déclenchement uniquement de l'arrêt de l'exhalation,
- déclenchement uniquement de l'arrêt de l'insufflation,
- absence de l'arrêt, soit de l'insufflation, soit de l'exhalation.

Dans un premier mode de réalisation, le déclenchement des mécanismes d'arrêt du cycle mécanique est défini sur un critère chronologique. Pour chaque cycle respiratoire, une activité musculaire détectée en continu avant et après un début d'un arrêt d'un état du cycle mécanique est réputée avoir activement déclenché ce mécanisme d'arrêt.

Dans un deuxième mode de réalisation, les informations sur les débuts et les fins des cycles mécaniques et sur l'activité des mécanismes d'arrêt du cycle sont fournies directement par l'appareil d'assistance. Dans ce cas, le système de quantification selon l'invention exploite une sortie de cet appareil qui fournit un signal représentatif de l'état des valves internes qui commandent l'insufflation et l'exhalation ainsi qu'un signal de déclenchement ou non de ces valves par une activité respiratoire du patient qui est alors détectée par des systèmes de mesure propres et intégrés à l'appareil d'assistance. Dans ce cas, la redondance des informations permet d'améliorer la robustesse du système.

Dans un troisième mode de réalisation, les informations sur les débuts et les fins des cycles respiratoires sont traitées avec des signaux de mesure de l'activité respiratoire additionnels au signal de débit et de pression. Il peut s'agir par exemple de signaux d'électromyographie des muscles respiratoires ou encore de pression intra thoracique (pression oesophagienne ou gastrique) qui sont donc conditionnés de façon appropriée par les moyens d'acquisition afin de détecter les débuts et les fins des cycles respiratoires. L'adjonction d'un signal complémentaire de mesure sur l'activité respiratoire permet d'améliorer la robustesse du traitement des signaux musculo-physiologiques pour caractériser les fins et les débuts du cycle respiratoire.

Une telle structure est illustrée sur la figure 5 sur laquelle le signal complémentaire est désigné par la référence générale 26.

Le calcul du score de désynchronisation peut être commun à ces différents modes de réalisation.

En fait, ce calcul exploite une distribution de probabilités des classes d'interactions observables pour le système global (N) et ses deux composants à savoir l'appareil d'assistance (C) et le patient (B).

Ce calcul prend en compte le mode de ventilation réglé sur l'appareil d'assistance par l'opérateur, dans la mesure où un mode peut être sélectionné parmi différents modes qui se distinguent par leur mécanisme d'arrêt du cycle mécanique. A titre d'exemple, 4 types de mode de ventilation peuvent être définis :

1) Mode volume contrôlé (VC) : aucun des mécanismes d'arrêt du cycle ne peut être déclenché par le patient,

2) Mode volume contrôlé assisté (VAC) : le patient ne peut déclencher que l'arrêt de l'exhalation,

3) Mode aide respiratoire (AI) : le patient peut déclencher indépendamment l'un de l'autre les deux mécanismes d'arrêt du cycle mécanique,

4) Mode d'assistance proportionnel (PAV) : le patient peut déclencher les deux mécanismes d'arrêt mais l'arrêt de l'insufflation ne peut être déclenché que si le mécanisme d'arrêt de l'exhalation a été préalablement activé.

Pour chaque mode, il est alors possible de construire, de façon classique, la distribution des classes d'interactions observables pour le système et ses deux composants, à savoir l'appareil et le patient.

Dans la suite de la description, b sera utilisé pour désigner le nombre de demandes patient observé, c le nombre de cycles mécaniques observé et n l'ensemble des événements observés pour le système patient-appareil.

En mode VC, le patient ne déclenche jamais l'appareil d'assistance de telle sorte qu'il n'existe que des activités inspiratoires non déclenchantes.

En mode AI, si l'on appelle r0 le nombre de cycles où les deux mécanismes d'arrêt on été activés par le patient, ri le nombre de cycles dont l'insufflation est arrêtée par le patient sans arrêt actif de l'exhalation, et re le nombre de cycles dont l'exhalation est arrêtée par le patient sans arrêt actif de l'insufflation, alors les nombres de cycles sans activation des mécanismes d'arrêt par le patient sont :

(b-(re + ri + r0)) pour les cycles respiratoires, et

(c-(re + ri + r0)) pour les cycles mécaniques.

La valeur de probabilité pour chaque classe et composant du système est estimée pour un temps d'observation donné, par le rapport entre le nombre d'événements de chaque classe et le nombre total d'événements du composant concerné. La probabilité de la i° classe du composant B, à savoir le patient, P(bi), est définie par le rapport entre le cardinal de la i° classe et le nombre d'événements observés pour la structure à savoir le nombre de cycles respiratoires. La probabilité pour l'ensemble du système, P(bi, cj), correspond à la valeur de probabilité pour la réalisation simultanée de l'événement de la i° classe du composant B et de celui de la j° classe du composant C. Par construction, le nombre d'événements des classes correspondant à la même combinaison d'activation des mécanismes d'arrêt du cycle est égal. Aussi, pour i différent de j, P(bi, cj) = 0 et pour i = j, P(bi, cj) est le ratio du nombre d'événements de la classe i ou j divisé par le nombre d'événements pour l'ensemble du système, c'est-à-dire n = b + c - (re + ri + r0).

A partir de l'estimation de cette distribution de probabilité, la quantité d'informations de chaque structure est donnée par la formule de l'entropie H de Shannon de façon classique. Pour le composant B, elle est donnée par l'expression suivante :

$$H(B) = -Si\ P(bi) * \log P(bi).$$

Pour un système N composé de deux composants B et C, l'entropie H de l'ensemble du système N est donnée par :

$$H(N) = -Si\ Sj\ P(bi, cj) * \log P(bi, cj).$$

Ici, comme par construction pour i différent de j, P(bi, cj) = 0, l'entropie du système global N est donnée par la relation :

$$H(N) = -Si\ Sj\ P(bi, cj) * \log P(bi, cj) = -Si\ P(bi, ci) * \log P(bi, ci) = -Si\ P(ni) * \log P(ni)$$

Le gain de quantité d'informations généré par la contrainte, autrement dit la qualité de la transmission établie entre les deux composants B et C du système N, est donnée par :

$$QI(N) = H(N) - [H(B) + H(C)]$$

Ce terme représente le score du désaccord entre le patient et l'appareil. Sa valeur est positive par construction.

Ce score fournit une quantification de l'importance du désaccord entre le patient et l'appareil et préserve les propriétés essentielles de l'entropie de Shannon.

Ce score prend des valeurs entre zéro et un maximum.

Sa valeur est à zéro en l'absence d'interaction (par exemple en VC où par définition l'appareil domine le patient et où il n'existe pas d'interaction) ou lorsque l'interaction est monotone avec une distribution de probabilité où seule une classe est représentée.

Dans la première situation, les deux composants fonctionnent de façon indépendante et la quantité d'informations du système H(N) est égale à la somme des quantités d'informations pour chaque composant de telle sorte que la valeur de QI est nulle.

Dans la deuxième situation, comme c'est le cas en mode AI ou en mode VAC, lorsque le patient déclenche de façon monotone et avec toujours le même type d'interaction, le système patient-appareil ne génère pas d'information et le score est égal à zéro. Dans le cas où à chaque inspiration correspond un cycle mécanique déclenché, H(B) = H(C) = H(N) = 0 et donc QI(N) = 0.

A l'inverse, si tous les types d'interactions sont observés, la quantité d'informations augmente jusqu'à un maximum. Cette limite est liée au maximum de l'entropie d'un système qui est atteint lorsque la distribution des N classes est homogène (Hmax = -Log (1/N)). Ces propriétés sont préservées quel que soit le nombre de classes définies et donc de mécanismes de déclenchement du cycle ou du mode de ventilation observé.

Pour sa validation clinique, le score a été calculé pour quantifier l'accord entre le patient et son appareil d'assistance sur le seul critère du déclenchement de l'insufflation mécanique.

Cette situation correspond au mode de ventilation partielle (Ventilation Assistée Contrôlée ou Aide Inspiratoire). Dans ce cas, il n'existe que trois situations, à savoir :

1) La demande patient déclenche l'appareil ;
2) La demande patient ne déclenche pas l'appareil ;
3) L'appareil se déclenche sans demande patient.

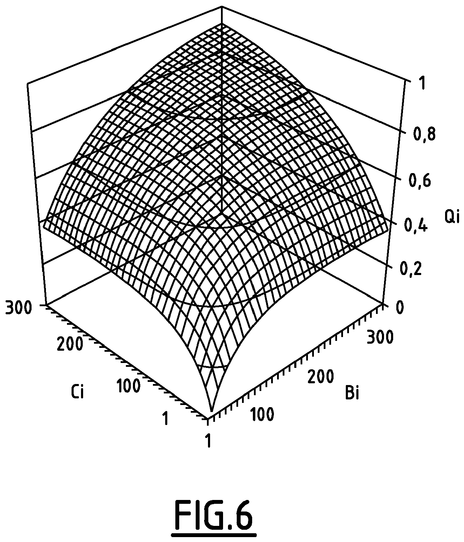

A titre d'exemple, la figure 6 illustre les variations du score QI avec le désaccord patient-appareil pour 200 cycles mécaniques déclenchés (Nt = re). La valeur de QI augmente avec l'importance du désaccord patient-appareil quantifié par le nombre de demandes inspiratoires non détectées et les auto-déclenchements des cycles mécaniques ($B_{nd}$ = (b-re) et $C_{ad}$ = (c-re) respectivement). La valeur de QI est à zéro en l'absence de tout désaccord patient-appareil ($C_{ad}$ = $B_{nd}$ = 0) et passe à la valeur 0,84 pour la situation où les nombres d'événements non équiprobables ($C_{ad}$ = $B_{nd}$ = $N_t$ = 200) pour atteindre asymptotiquement la valeur de 1,369 :

- QI est minimum (QI = 0.0) en l'absence de tout désaccord patient-appareil ($C_{ad}$ = $B_{nd}$ = 0)
- QI augmente régulièrement avec le nombre d'événements ($C_{ad}$ ou $B_{nd}$). QI tend asymptotiquement vers un maximum lorsque les événements sont équiprobables ($C_{ad}$ = $B_{nd}$) et suffisamment élevés pour que $N_t$ soit négligeable devant $C_{ad}$ ou $B_{nd}$. Dans ce cas, P($C_{ad}$) = P($B_{nd}$) = 0,5 d'où H(C)= H(B) = 1 et H(N) = 0,631 avec QI = 1,369.

De même, pour une valeur donnée de $C_{ad}$ ou de $B_{nd}$, QI augmente pour atteindre un maximum pour $B_{nd}$ = $N_t$ = 200 ou $C_{ad}$. Au-delà de ce maximum, QI reste élevé et proche de ce maximum.

On conçoit alors que le score peut être affiché sous une forme numérique ou graphique pour être perçu très simplement et facilement par l'opérateur, afin d'engager si nécessaire un processus correctif.

Il va de soi bien entendu que d'autres modes de réalisation encore peuvent être envisagés.

**Revendications**

**1.** Système de quantification du désaccord entre un patient sous assistance respiratoire et un appareil d'assistance correspondant, comportant :

- des moyens (20) d'acquisition d'informations relatives à l'état respiratoire du patient (1) sur un cycle respiratoire,
- des moyens (21) d'acquisition d'informations relatives à l'état de fonctionnement de l'appareil d'assistance (2) sur un cycle mécanique,
- des moyens de filtrage (22) et de traitement (23) de ces informations pour détecter et localiser les changements d'états du patient sur un cycle respiratoire et de l'appareil sur un cycle mécanique,
- des moyens (24) de calcul d'une information de désynchronisation entre le patient et l'appareil à partir de ces changements d'états, et
- des moyens (25) formant interface homme/machine de restitution de cette information de désynchronisation à un opérateur, **caractérisé en ce que** les moyens d'acquisition des informations relatives à l'état respiratoire du patient comprennent des moyens (4, 5) d'acquisition de signaux de pression et de débit de l'air dans un circuit pneumatique (3) entre le patient (1) et l'appareil d'assistance (2), à destination de moyens (6) d'estimation en continu de la pression théorique de l'air attendue dans le circuit pneumatique (3) en l'absence d'activité musculaire respiratoire du patient et des moyens (7) de comparaison des pressions théorique estimée et mesurée pour détecter en continu une différence de pression représentative d'une activité musculaire respiratoire du patient.

**2.** Système de quantification selon la revendication 1, **caractérisé en ce que** les moyens (21) d'acquisition d'informations relatives à l'état de fonctionnement de l'appareil d'assistance comprennent des moyens de traitement d'un signal de débit d'air de l'appareil pour détecter les états d'exhalation et d'insufflation de celui-ci.

**3.** Système de quantification selon les revendications 1 et 2, **caractérisé en ce que** les moyens (21) d'acquisition d'informations relatives à l'état de fonctionnement de l'appareil d'assistance sont intégrés dans celui-ci.

**4.** Système de quantification selon la revendication 2, **caractérisé en ce que** les moyens d'acquisition d'informations relatives à l'état de fonctionnement de l'appareil d'assistance comprennent un capteur de débit d'air associé au circuit pneumatique (3) entre l'appareil d'assistance (2) et le patient (1).

**5.** Système de quantification selon l'une quelconque des revendications précédentes, **caractérisé en ce que** les moyens (24) de calcul de l'information de désynchronisation comprennent des moyens de calcul d'un score de désaccord patient/machine selon la relation :

$$QI(N) = H\,(N) - [H(B) + H(C)]$$

dans laquelle QI(N) est le score de désynchronisation, H(N) est l'entropie de Shannon du système appareil d'assistance (2) + patient (1), H(B) est l'entropie de Shannon du patient seul (1) et H(C) est l'entropie de Shannon de l'appareil d'assistance seul (2).

**6.** Système de quantification selon la revendication 5, **caractérisé en ce que** les moyens (25) formant interface homme/machine comprennent des moyens d'affichage de ce score.

**7.** Système de quantification selon la revendication 6, **caractérisé en ce que** les moyens d'affichage (25) comprennent des moyens d'affichage de ce score sous forme numérique.

**8.** Système de quantification selon la revendication 6, **caractérisé en ce que** les moyens d'affichage (25) comprennent des moyens d'affichage de ce score sous forme graphique.

**9.** Système de quantification selon l'une quelconque des revendications précédentes, **caractérisé en ce que** les moyens de calcul (24) sont adaptés pour calculer l'information de désynchronisation sur un nombre de cycles respiratoires prédéterminé.

**10.** Système de quantification selon la revendication 1 et l'une quelconque des revendications 2 à 9, **caractérisé en ce que** les moyens (6) d'estimation comprennent des moyens (8) paramétrables et adaptatifs de modélisation du système respiratoire passif du patient.

**11.** Système de quantification selon la revendication 10, **caractérisé en ce que** les moyens (8) de modélisation se présentent sous la forme de modèles fonction au moins du volume et du débit d'air circulant dans le circuit pneumatique.

**12.** Système de quantification selon la revendications 10 ou 11, **caractérisé en ce que** les moyens de modélisation comprennent un ensemble (8) de modèles paramétrables et **en ce que** les moyens d'estimation comprennent des moyens (9) d'extraction du signal de pression mesurée, de paramètres d'entrée de ces modèles, de façon à déclencher le fonctionnement de ces modèles sur la base de ces paramètres et des moyens (10) de sélection du modèle le plus discriminant en terme de détection et de non-détection d'activité musculaire respiratoire du patient et/ou le plus simple en terme de nombre de paramètres utilisés, pour retenir son estimation.

**13.** Système de quantification selon la revendication 12, **caractérisé en ce que** les moyens (9) d'extraction des paramètres sont adaptés pour extraire les paramètres sur un cycle mécanique composé successivement d'une insufflation et d'une exhalation, en excluant la phase (11) de pressurisation au début du cycle mécanique courant et la phase (12) de déclenchement de l'insufflation du cycle suivant, à la fin du cycle mécanique courant.

14. Système de quantification selon la revendication 13, **caractérisé en ce que** la phase (11) de pressurisation et la phase (12) de déclenchement de l'insufflation sont détectées par des moyens d'analyse de la pression et du débit d'air dans le circuit pneumatique (3).

15. Système de quantification selon la revendication 14, **caractérisé en ce que** les moyens d'analyse sont raccordés aux moyens (4, 5) d'acquisition des signaux de pression et de débit dans le circuit pneumatique (3).

16. Système de quantification selon la revendication 14 ou 15, **caractérisé en ce que** les moyens d'analyse sont intégrés dans l'appareil d'assistance (2).

17. Système de quantification selon la revendication 13, **caractérisé en ce que** les phases (11) de pressurisation et (12) de déclenchement de l'insufflation sont détectées par des moyens d'analyse à partir d'un signal complémentaire (14) délivrant une information physiologique liée à l'activité musculaire respiratoire du patient.

18. Système de quantification selon la revendication 17, **caractérisé en ce que** le signal complémentaire est un signal d'électromyogramme.

**Patentansprüche**

1. System zur Quantifizierung der Diskrepanz zwischen einem Patienten mit Atemunterstützung und einer entsprechenden Unterstützungsvorrichtung, aufweisend:

   - Mittel (20) für die Erfassung von Informationen über den Beatmungszustand des Patienten (1) für einen Atemzyklus,
   - Mittel (21) für die Erfassung von Informationen über den Funktionszustand der Unterstützungsvorrichtung (2) für einen mechanischen Zyklus,
   - Filter- (22) und Verarbeitungsmittel (23) dieser Informationen, um Zustandsänderungen des Patienten für einen Atemzyklus und der Vorrichtung für einen mechanischen Zyklus festzustellen und zu lokalisieren,
   - Berechnungsmittel (24) einer Desynchronisierungsinformation zwischen dem Patienten und der Vorrichtung auf der Basis dieser Zustandsänderungen, und
   - Mittel (25), die eine Mensch-Maschine-Schnittstelle für die Bereitstellung dieser Desynchronisierungsinformation an eine Bediener,

   **dadurch gekennzeichnet, dass** die Mittel für die Erfassung von Informationen über den Beatmungszustand des Patienten Erfassungsmittel (4, 5) von Luftdruck-und Durchsatzinformationen in einem Pneumatikkreis (3) zwischen dem Patienten (1) und der Unterstützungsvorrichtung (2), bestimmt für Mittel (6) zur kontinuierlichen Schätzung des erwarteten theoretischen Luftdrucks im Pneumatikkreis (3) bei Abwesenheit von Atemmuskelaktivität des Patienten, und Vergleichsmittel (7) des theoretisch geschätzten und gemessenen Drucks umfasst, um kontinuierlich eine Druckdifferenz festzustellen, die für eine Atemmuskelaktivität des Patienten repräsentativ ist.

2. System zur Quantifizierung nach Anspruch 1, **dadurch gekennzeichnet, dass** die Mittel (21) für die Erfassung von Informationen über den Funktionszustand der Unterstützungsvorrichtung Verarbeitungsmittel eines Luftdurchsatzsignals der Vorrichtung umfassen, um Aus- und Einatemzustände derselben festzustellen.

3. System zur Quantifizierung nach den Ansprüchen 1 und 2, **dadurch gekennzeichnet, dass** die Mittel (21) für die Erfassung von Informationen über den Funktionszustand der Unterstützungsvorrichtung in diese integriert sind.

4. System zur Quantifizierung nach Anspruch 2, **dadurch gekennzeichnet, dass** die Mittel für die Erfassung von Informationen über den Funktionszustand der Unterstützungsvorrichtung einen Luftdurchsatzsensor umfassen, der dem Pneumatikkreis (3) zwischen der Unterstützungsvorrichtung (2) und dem Patienten (1) zugeordnet ist.

5. System zur Quantifizierung nach einem der vorangehenden Ansprüche, **dadurch gekennzeichnet, dass** die Berechnungsmittel (24) der Desynchronisierungsinformation Berechnungsmittel eines Diskrepanzscores Patient/Maschine gemäß der Gleichung

$$QI(N) = H(N) - [H(B) + H(C)]$$

umfassen, wobei QI(N) der Desynchronisierungsscore ist, H(N) die Shannon-Entropie des Systems Unterstützungsvorrichtung (2) + Patient (1) ist, H(B) die Shannon-Entropie nur des Patienten (1) ist und H(C) die Shannon-Entropie nur der Unterstützungsvorrichtung (2) ist.

6. System zur Quantifizierung nach Anspruch 5, **dadurch gekennzeichnet, dass** die Mittel (25), die die Mensch-Maschine-Schnittstelle bilden, Anzeigemittel dieses Score umfassen.

7. System zur Quantifizierung nach Anspruch 6, **dadurch gekennzeichnet, dass** die Anzeigemittel (25) Anzeigemittel dieses Score in digitaler Form umfassen.

8. System zur Quantifizierung nach Anspruch 6, **dadurch gekennzeichnet, dass** die Anzeigemittel (25) Anzeigemittel dieses Score in grafischer Form umfassen.

9. System zur Quantifizierung nach einem der vorangehenden Ansprüche, **dadurch gekennzeichnet, dass** die Berechnungsmittel (24) ausgebildet sind, um die Desynchronisierungsinformation für eine vorbestimmte Anzahl von Atemzyklen zu berechnen.

10. System zur Quantifizierung nach Anspruch 1 und einem der Ansprüche 2 bis 9, **dadurch gekennzeichnet, dass** die Schätzmittel (6) parametrierbare und adaptive Modellierungsmittel (8) des passiven Atemsystems des Patienten umfassen.

11. System zur Quantifizierung nach Anspruch 10, **dadurch gekennzeichnet, dass** die Modellierungsmittel (8) in Form von Funktionsmodellen mindestens des Luftvolumens und -durchsatzes vorliegen, das / der im Pneumatikkreis zirkuliert.

12. System zur Quantifizierung nach Anspruch 10 oder 11, **dadurch gekennzeichnet, dass** die Modellierungsmittel (8) eine Gruppe von parametrierbaren Modellen umfassen und dass die Schätzmittel Extraktionsmittel (9) des Signals des gemessenen Drucks, von Eingangsparametern dieser Modelle, umfassen, um die Funktion dieser Modelle auf der Basis von diesen Parametern auszulösen, und Selektionsmittel (10) des diskriminierendsten Modells im Hinblick auf Ermittlung und nicht Ermittlung von Atemmuskelaktivität des Patienten und/oder des einfachsten Modells im Hinblick auf die Anzahl der verwendeten Parameter, um seine Schätzung zu behalten.

13. System zur Quantifizierung nach Anspruch 12, **dadurch gekennzeichnet, dass** die Extraktionsmittel (9) der Parameter ausgebildet sind, um die Parameter aus einem mechanischen Zyklus zu extrahieren, der sich aufeinanderfolgend aus einem Einatmen und einem Ausatmen zusammensetzt, bei Ausschluss der Druckbeaufschlagungsphase (11) zu Beginn des laufenden mechanischen Zyklus und der Phase (12) des Auslösens des Einatmens des folgenden Zyklus am Ende des laufenden mechanischen Zyklus.

14. System zur Quantifizierung nach Anspruch 13, **dadurch gekennzeichnet, dass** die Druckbeaufschlagungsphase (11) und die Phase (12) des Auslösens des Einatmens von Analysemitteln des Luftdrucks und -durchsatzes im Pneumatikkreis (3) festgestellt werden.

15. System zur Quantifizierung nach Anspruch 14, **dadurch gekennzeichnet, dass** die Analysemittel mit Erfassungsmitteln (4, 5) von Druck- und Durchsatzsignalen im Pneumatikkreis (3) gekoppelt sind.

16. System zur Quantifizierung nach Anspruch 14 oder 15, **dadurch gekennzeichnet, dass** die Analysemittel in die Unterstützungsvorrichtung (2) integriert sind.

17. System zur Quantifizierung nach Anspruch 13, **dadurch gekennzeichnet, dass** die Phasen der Druckbeaufschlagung (11) und des Auslösens des Einatmens (12) von Analysemitteln mit Hilfe eines komplementären Signals (14) festgestellt werden, da seine physiologische Information in Verbindung mit der Atemmuskelaktivität des Patienten bereitstellt.

18. System zur Quantifizierung nach Anspruch 17, **dadurch gekennzeichnet, dass** das komplementäre Signal ein Elektromyogrammsignal ist.

**Claims**

1. A system for quantifying the discrepancy between a patient receiving breathing assistance and a corresponding assistance device, comprising:

   - means (20) for acquiring information relating to the respiratory state of a patient (1) over a breathing cycle,
   - means (21) for acquiring information relating to the operating state of the assistance device (2) over a mechanical cycle,
   - means for filtering (22) and processing (23) this information in order to detect and localize changes in states of the patient over a breathing cycle and of the device over a mechanical cycle,
   - means (24) for calculating a piece of desynchronization information between the patient and the device from these changes in states, and
   - means (25) forming a man/machine interface for returning this piece of desynchronization information to an operator, **characterized in that** the means for acquiring information relating to the respiratory state of a patient comprise means (4, 5) for acquiring air pressure and flow rate signals in a pneumatic circuit (3) between the patient (1) and the assistance device (2), intended for means (6) for continuously estimating the expected theoretic pressure of the air in the pneumatic circuit (3) in the absence of respiratory muscle activity of the patient, and means (7) for comparing the estimated and measured theoretical pressure in order to continuously detect a pressure difference representative of respiratory muscle activity of the patient.

2. The quantification system according to claim 1, **characterized in that** the means (21) for acquiring information relating to the operating state of the assistance device comprise means for processing an air flow rate signal of the device in order to detect the exhalation and insufflation states of the latter.

3. The quantification system according to claims 1 and 2, **characterized in that** the means (21) for acquiring information relating to the operating state of the assistance device are integrated into the latter.

4. The quantification system according to claim 2, **characterized in that** the means for acquiring information relating to the operating state of the assistance device comprise an air flow rate sensor associated with the pneumatic circuit (3) between the assistance device (2) and the patient (1).

5. The quantification system according to any of the preceding claims, **characterized in that** the means (24) for calculating the piece of desynchronization information comprise means for calculating a patient/machine discrepancy score according to the relationship:

$$QI(N) = H(N) - [H(B) + H(C)]$$

   wherein QI(N) is the desynchronization score, H(N) is the Shannon entropy of the assistance device (2) + patient (1)) system, H(B) is the Shannon entropy of the patient alone (1) and H(C) is the Shannon entropy of the assistance device (2) alone.

6. The quantification system according to any claim 5, **characterized in that** the means (25) forming a man/machine interface comprise means for displaying this score.

7. The quantification system according to claim 6, **characterized in that** the display means (25) comprise a means for displaying this score in digital form.

8. The quantification system according to claim 6, **characterized in that** the display means (25) comprise means for displaying this score in graphic form.

9. The quantification system according to any of the preceding claims, **characterized in that** the calculation means (24) are adapted for calculating the piece of desynchronization information over a predetermined number of breathing cycles.

**10.** The quantification system according to claim 1 and to any of claims 2 to 9, **characterized in that** the estimation means (6) comprise parameterizable and adaptive means (8) for modeling the passive respiratory system of the patient.

**11.** The quantification system according to claim 10, **characterized in that** the modeling means (8) appear as models at least depending on the volume and on the flow of air circulating in the pneumatic circuit.

**12.** The quantification system according to claim 10 or ,11 **characterized in that** the modeling means comprise a set (8) of parameterizable models and **in that** the estimation means comprise means (9) for extracting the measured pressure signal, input parameters of these models, so as to trigger the operation of these models on the basis of these parameters and means (10) for selecting the most discriminating model in terms of detection and nondetection of respiratory muscle activity of the patient and/or the simplest model in terms of the number of parameters used, in order to retain its estimation.

**13.** The quantification system according to claim 12, **characterized in that** the means (9) for extracting the parameters are adapted so as to extract the parameters over at least one mechanical cycle successively consisting of insufflation and exhalation, while excluding the pressurization phase (11) at the beginning of the current mechanic cycle and the phase (12) for triggering the insufflation of the next cycle, at the end of the current mechanical cycle.

**14.** The quantification system according to claim 13, **characterized in that** the pressurization phase (11) and the phase (12) for triggering insufflation are detected by means for analyzing the air pressure and flow rate in the pneumatic circuit (3).

**15.** The quantification system according to claim 14, **characterized in that** the analysis means are connected to the means (4, 5) for acquiring pressure and flow rate signals in the pneumatic circuit (3).

**16.** The quantification system according to claim 14 or 15, **characterized in that** the analysis means are integrated into the assistance device (2).

**17.** The quantification system according to claim 13, **characterized in that** the phases (11) for pressurization and (12) for triggering the insufflation are detected by analysis means from a complementary signal (14) delivering a piece of physiological information related to the respiratory muscle activity of the patient.

**18.** The quantification system according to claim 17, **characterized in that** the complementary signal is an electromyogram signal.

## FIG.1

## FIG.2

## FIG.3

**FIG.4**

**FIG.5**

## FIG.6

**EP 2 364 174 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004002561 A